Europäisches Patentamt

European Patent Office

Office européen des brevets

(19)

(11) **EP 1 226 864 B1**

(12) **EUROPEAN PATENT SPECIFICATION**

(45) Date of publication and mention
of the grant of the patent:
**31.08.2005 Bulletin 2005/35**

(51) Int Cl.⁷: **B01F 17/00**, A61K 7/00

(21) Application number: **01113965.6**

(22) Date of filing: **08.06.2001**

(54) **A stable water-in-oil-in-water multiple emulsion system by hydrodynamic dual stabilization and a method for preparation thereof**

Stabile Wasser/oel/wasser-Emulsion erhalten durch hydrodynamische duale stabilisierung und Verfahren zu deren Herstellung

Emulsion multiple eau/huile/eau obtenue par double stabilisation hydrodynamique et procédé de préparation

(84) Designated Contracting States:
**AT BE CH CY DE DK ES FI FR GB GR IE IT LI LU MC NL PT SE TR**

(30) Priority: **29.12.2000 KR 2000085163**

(43) Date of publication of application:
**31.07.2002 Bulletin 2002/31**

(73) Proprietor: **PACIFIC CORPORATION**
**Seoul 140-777 (KR)**

(72) Inventors:
• **Kim, Jin Woong**
**Yongin-city, Kyungki-do (KR)**
• **Lee, Sung Il**
**Songpa-ku, Seoul (KR)**
• **Chae, Byung Guen**
**Songpa-ku, Seoul (KR)**
• **Kim, Han Kon**
**Suwon-city, Kyungki-do (KR)**
• **Kang, Hak Hee**
**(Satbyul-maul), Bundang-ku, Kyungki-do (KR)**
• **Jang, Li Sub**
**Suji-up, Yongin-city, Kyungki-do (KR)**

(74) Representative: **Maisch, Thomas**
**Ullrich & Naumann,**
**Patent- und Rechtsanwälte,**
**Luisenstrasse 14**
**69115 Heidelberg (DE)**

(56) References cited:
**EP-A- 0 345 075          EP-A- 0 958 856**
**DE-A- 19 612 084         FR-A- 2 766 737**
**US-A- 4 254 105          US-A- 5 332 595**
**US-A- 5 478 561**

**Description**

**BACKGROUND OF THE INVENTION**

**Field of the Invention**

The present invention relates to a water-in-oil-in-water multiple emulsion system maximizing stability in the internal aqueous phase of the multiple emulsion system and a method for preparing the same. In particular, the present invention relates to a method for preparing a water-in-oil-in-water multiple emulsion system with highly improved stability achieved through the following steps: 1) hydrophobizing (processing hydrophilic materials so as to change it into hydrophobic materials) internal aqueous phase by using hydrodynamic dual stabilization (hereinafter, refers to 'HDS') technology developed by the present invention in order to maximize dispersion stability that the internal phase stably interacts with the oil phase of the multiple emulsion; and, 2) re-emulsifying the result in order to block the outflow of the internal aqueous phase domain to the external aqueous phase in the obtained water-in-oil-in-water multiple emulsion system.

**Description of the Related Arts**

The multiple emulsions are divided into water-in-oil-in-water and oil-in-water-in-oil forms. In consideration of practical use, water-in-oil-in-water emulsion has been more widely applied to various fields. In drug delivery systems introducing water-soluble active materials into an internal aqueous phase for releasing in target sites from the internal aqueous phase chemically or physically, the water-in-oil-in-water emulsion attracts much interest because this multiple emulsion is a unique system in which the water domain is formed inside the oil phase of multiple emulsion system.

Until recently, water-in-oil-in-water multiple emulsion was prepared by a phase-transition method or two-step consecutive emulsification method. The phase-transition method using bifunctional solubility of the surfactant itself is applied broadly in the early stages, however, is limited in use because an unstable multiple emulsion is prepared by this method. Therefore, a two-step consecutive emulsification method for improving stability has been recently developed. This two-step consecutive emulsification method comprises the following two steps: 1) preparing water-in-oil emulsion by using hydrophobic surfactants; and, 2) re-emulsifying water-in-oil emulsion obtained in the above with water phase containing a suitable amount of hydrophilic surfactants.

When applying the two-step consecutive emulsification method, mixing ratio of surfactants, oil properties, mechanical shearing force, volume ratio of phase and introduction of water-soluble additives should be considered. Also, suitable surfactants should be chosen for preparing a stable multiple emulsion. It is commonly known that HLB (hydrophilic lypophilic balance) value of surfactants is preferably 3~7 in water-in-oil emulsification, and is preferably 8~16 in water-in-oil-in-water emulsification. However, selecting suitable surfactant is also dependent on oil properties, thus the HLB value range of selected surfactant may be changed by some kinds of systems.

Further, when preparing multiple emulsion by selecting suitable surfactants, factors including mechanical shearing force and volume ratio of phase affect droplet size, dispersibility, yield of multiple emulsion, phase morphology of the finally obtained multiple emulsion. Suitable conditions of these factors should be adjusted experimentally, depending on the applied fields. Water-soluble materials added to the internal or external aqueous phase of a multiple emulsion system may reduce or enhance stability of the multiple emulsion system. This effect of water-soluble materials results from concentration gradients formed by additives. Therefore, when applying to drug delivery systems by introducing water-soluble materials into the internal aqueous phase, suitable non-active water-soluble materials should be added in order to attenuate the concentration difference between the internal and external aqueous phases formed by the active material itself in the aqueous phases.

As mentioned above, stability of a multiple emulsion system can be improved only within a narrow range by changes of thermodynamic phase composition. Therefore, a system with improved stability should be devised in consideration of mutual relations of all factors affecting stability of the multiple emulsion system.

Under these circumstances, the present inventors have conducted much research to improve the stability of multiple emulsion systems. As a result, it is found that water molecules can be stabilized hydrodynamically by hydrophobizing water molecules in internal aqueous phase by using a hydrogen bonding inhibitor, and by improving aggregating force between water molecules using a water molecule aggregating agent. The water-in-oil-in-water multiple emulsion systems obtained by hydrodynamical stabilization of the water molecules are highly stable.

US-A-4 254 105 discloses a water-in-oil-in-water multiple emulsion system which is kinetically stabilized by using an agent for aggregating the water molecules selected from the group of oligosaccharides.

EP-A-0 958 856 also discloses a kinetically stabilized water-in-oil-in-water triple emulsion comprising an aqueous external phase and a water-in-oil primary emulsion comprising an oily phase and an aqueous internal phase, whereby the oily phase comprises at least one partially or completely crosslinked organopolysiloxane elastomer comprising a polyoxyethylenated and/or polyoxypropylenated chain, used in a topically composition with a medically or cosmetically active ingredient.

FR-A-2 766 737 discloses a multiple emulsion system comprising a lipophilic and a hydrophilic part by using vegetable lecithins and/or synthetic lecithins or fractionated lecithins. This formed emulsion is used for cosmetic compositions, pharmaceutical compositions or is applied as a food.

DE-A-196 12 084 discloses a method for preparing water-in-oil-in-water multiple emulsion system with an oily phase containing an emulgator mixture comprising polyolpoly-12-hydroxystearate, alcyl- and/or alcenyloligoglycoside and fatty alcohol and/or partially glyceride.

## SUMMARY OF THE INVENTION

Thus, an object of the present invention is to provide a multiple emulsion system with much-improved stability compared to the conventional multiple emulsion systems.

Another object of the present invention is to provide a method for preparing said multiple emulsion system with improved stability.

To achieve the above object, there is provided a water-in-oil-in-water multiple emulsion system according to claim 1 and method for preparing the water-in-oil-in-water multiple emulsion system according to claim 2.

## DETAILED DESCRIPTION OF THE INVENTION

The present invention is explained in detail below.

Water is a typical example of materials with high polarity. Further, water in the internal aqueous phase of multiple emulsion system shares the same high polarity with water in the external aqueous phase. Therefore, improvement of stability by the conventional method reaches limits because water in the internal aqueous phase strongly intends to contact with the water in the external aqueous phase, although surfactants are added and phase conditions are controlled.

The present inventors were interested in whether said problems caused by water polarity can be overcome or not when polar properties of water in internal or external phase can be changed.

According to the present invention, compatibility of the internal aqueous phase with the oil phase can be improved, and the attributes intending to be combined with water in external phase can be reduced by hydrodynamical hydrophobization of water in the internal aqueous phase. As a result of these effects, the finally obtained multiple emulsion system possesses excellent stability in the internal aqueous phase.

Therefore, the HDS technology according to the present invention is based on hydrophobization of the water in the internal phase and minimization of its fluidity. These purposes are achieved by introducing hydrogen bonding inhibitor and water molecule aggregating agent into the water in the internal aqueous phase.

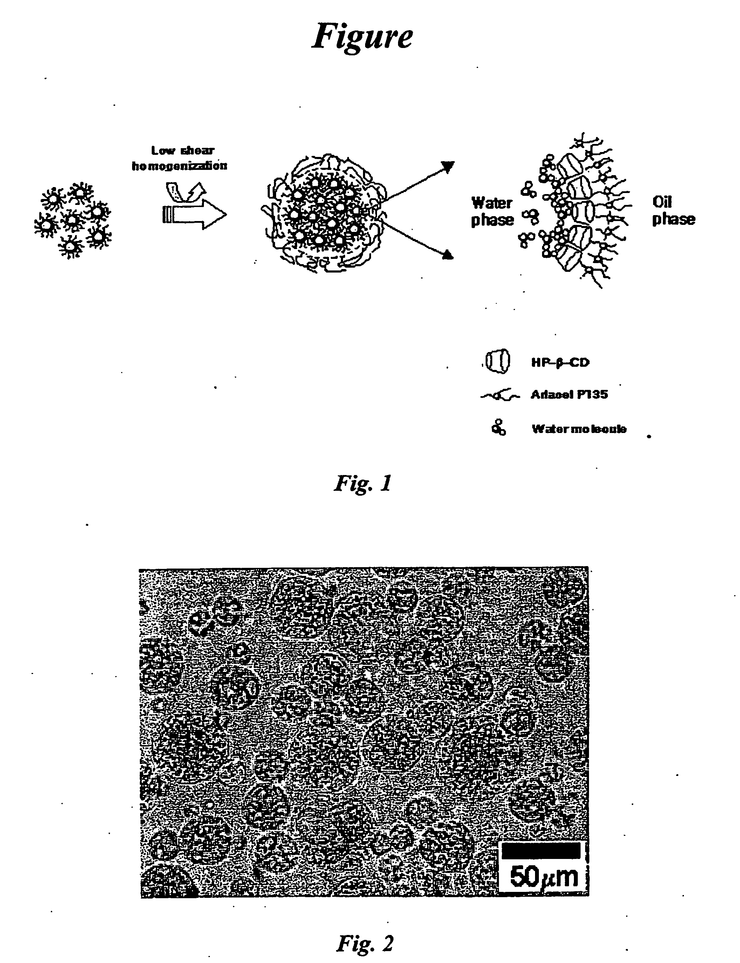

Polarity of water is facilitated by dissymmetrical linkage of two hydrogen atoms and one oxygen atom inside a water molecule and strong hydrogen bonding among water molecules. Polarity of water may be reduced by introducing hydrogen bonding inhibitor. Water molecules with reduced polarity may be changed to macromolecule form by secondary treatment with a water molecule aggregating agent. Therefore, the water phase is hydrophobized and its fluidity is minimized by treatment with said hydrogen bonding inhibitor and aggregating agent. These procedures are explained in the Schematic Figure 1.

When the water phase treated with the HDS is positioned onto the internal phase of a multiple emulsifying system, the stability of emulsion system is maximized for the following reasons.

First, hydrophobized water phase may increase in compatibility with the oil phase. Unlike the conventional methods for simply decreasing in interfacial tension by using surfactants, the hydrophobization of the water phase of the invention is a procedure for improving compatibility between two interfaces through increasing in natural affinity of water phase to oil phase. As a result, compatibility between the water of internal phase and the oil phases becomes improved. Therefore, upon the primary preparation of a water-in-oil emulsion, extremely small water domains may be formed, thus this procedure contributes to improvement of stability of water-in-oil emulsion itself.

Second, diffusion of water molecules into oil is decreased because the fluidity of water existing in the internal phase of a water-in-oil-in-water emulsion prepared through the secondary emulsifying procedure is minimized. Therefore, the present invention solves problem that the interface is weakened by outflow into external phase. As a result, the stability of multiple emulsion system is improved excessively.

Third, the present invention does not need to use other additives for improving the stability of multiple emulsion. Therefore, problems caused by water-soluble active materials may be solved in practical uses such as drug delivery systems.

The water phase, stabilized hydromechanically by using a hydrogen bonding inhibitor and a water molecule aggregating agent, is evenly mixed with hydrophobic surfactant at a temperature higher than the melting points of the added two agents. Then, a stable water-in-oil emulsion is prepared by a strong mechanical shearing force. The term 'shearing force' means external force for emulsifying through shearing and mixing particles in an aqueous phase or an oil phase with mechanical force. A typical example of emulsifier is homogenizer. The homogenizer controls particle sizes, thus affecting the degree of emulsification.

According to the present invention, the amount of hydrogen bonding inhibitor is preferably 0.01~5% by weight based on the total weight of the water-in-oil-in-water multiple emulsion system. It is preferable that the hydrogen bonding inhibitor has a mutual attraction in two or more directions on the hydrogen atom or oxygen atom in the molecule. Examples of hydrogen bonding inhibitor include all water-soluble molecules such as urea, thio-urea, alcohols and amines with low molecular weights.

It is preferable that water molecule aggregating agents have ability collecting water molecules, and examples of such agents include cyclodextrine and its derivatives and analogues. Amount of aggregating agent employed for the present invention is preferably 0.01~5% by weight based on the total weight of the water-in-oil-in-water multiple emulsion system.

Also, the amount of hydrophobic surfactant is preferably 0.01~10% by weight based on the total weight of the water-in-oil-in-water multiple emulsion system. Hydrophobic surfactant is selected from all hydrophobic surfactants having the HLB value within the range of 1~7.

Further, the water-in-oil-in-water multiple emulsion is prepared by re-emulsifying water-in-oil emulsion obtained under said emulsification conditions, with low mechanical shearing force, in aqueous phase dissolving in hydrophilic surfactant.

According to the present invention, hydrophilic surfactant is preferably 0.01~10% by weight based on the total weight of the water-in-oil-in-water multiple emulsion system. Examples of such surfactants include all non-ionic surfactants within the HLB value range of 8~20 or all ionic surfactants within the HLB value of 20 or more. Also, a composition of mixture surfactants may be employed for the present invention.

Finally, according to the present invention, a dispersion stabilizing agent may be added to said obtained multiple emulsion in order to increase the viscosity of the external aqueous phase, resulting in much-improved stability of multiple emulsion. The dispersion stabilizing agent employed in the present invention is preferably a macromolecules capable of being dissolved in aqueous phase. Examples of such dispersion stabilizing agents includes gelatin, starch, xanthan gun, sodium polyacrylate, hydroxyethyl cellulose, carboxymethyl cellulose, polypyrrolidone, polyvinyl alkyl ether, polyvinyl alcohol, random or block copolymer conjugating hydrophilic group to main chain and the like.

The water-soluble active materials contained in the internal aqueous phase of water-in-oil-in-water multiple emulsion system according to the present invention, include any active materials employed in cosmetics, for example, water-soluble ingredients of functional cosmetics including vitamin B, vitamin C and their derivatives; peptides compounds and proteins; enzymes; water-soluble plant extracts and active materials including the same; water-soluble functional active extracts and the like.

These water-soluble functional active materials are highly unstable, thus may be stabilized by introduction into the internal aqueous phase of water-in-oil-in-water multiple emulsion system. Therefore, one purpose of preparing the water-in-oil-in-water multiple emulsion is to stabilize these water-soluble functional active materials.

Also, the water-in-oil-in-water multiple emulsion system may be applied to drug delivery systems. In drug delivery systems, introducing water-soluble drugs into water-in-oil-in-water multiple emulsion system may result in stabilization of drugs and controlled release rate of drugs. Therefore, water-soluble materials employed in drug delivery systems as well as cosmetics may be stabilized and their release rate may be controlled with water-in-oil-in-water multiple emulsion system structure.

When an active drug material of medical composition is water-soluble, it is included in the internal aqueous phase of a water-in-oil-in-water multiple emulsion system. These active drug materials include amino acids and water-soluble antibiotics. Amino acids are used as injection materials. Water-soluble antibiotics include macrolide-family antibiotics such as tetracycline HCl, minocyclin HCl, ampicillin, zentamycin HCl. These water-soluble antibiotics are used for primary treatment of infectious diseases, however, these antibiotics have a short half-life period and are released fast, thus a slow release property is necessary for effective use of these antibiotics. Therefore, the present invention also provides these antibiotics with a slow-release property.

## BRIEF DESCRIPTION OF DRAWINGS

Figure 1 is a schematic drawing of the interface in water-in-oil-in-water multiple emulsion system obtained by HDS (hydrodynamic dual stabilization).

Figure 2 is an optical microscope image observing the water-in-oil-in-water multiple emulsion obtained in the conventional method.

Figure 3 is optical microscope image observing water-in-oil-in-water multiple emulsion obtained by HDS (adding 0.5wt% urea by weight, 1wt% hydroxypropyl-beta-cyclodextrine).

Figure 4 is optical microscope image observing water-in-oil-in-water multiple emulsion of which 1wt% kojic acid by weight is contained and stabilized in an internal aqueous phase.

**PREFERRED EMBODIMENT OF THE INVENTION**

In more detail, the present invention is explained by preferred embodiments, comparative examples and experimental examples. However, the present invention should not be limited to these examples.

Example 1: Preparation of multiple emulsion according to the present invention

A multiple emulsion stabilized by HDS was prepared by carrying out the following procedures. After hydrophobizing water by adding 1wt% of urea and 2wt% of hydroxypropyl-beta-cyclodextrine (NIPPON Food Chemical Engineering Co., Ltd.), 30wt% of the hydrophobized water was melted, stirring gently at a temperature of 70~75°C (Mixture A). The degree of hydrophobicity of water was determined by measuring the interface tension of the water. Then, 15wt% of mineral oil (LP70™, Witco Co., U.S.) dissolving 1.5wt% of hydrophobic surfactant and PEG-30 dipolyhydroxystearate (Arlacel P135™, ICI Co., GB) were melted, stirring gently at a temperature of 70~75°C (Mixture B). Then, mixture A and B were mixed violently for 5 minutes using homogenizer of 7,000~8,000rpm. After mixing, results were cooled at 50~60°C, thus a highly stable water-in-oil emulsion was obtained.

Next, the water phase dissolved in 0.5wt% of poloxamer 407 (Synperonic PE/F 127™, ICI Co.), was added slowly to water-in-oil emulsion obtained in the above procedure, stirring with homogenizer of 4,000rpm at 50~60°C. Herein, the concentration was fixed to 50wt%. Then, 1wt% of xanthan gum solution (Kelfrol-F™, Kelco Co., U.S.) was added at amount of 10wt%. Thus-obtained water-in-oil-in-water multiple emulsion was stabilized, then cooled slowly at room temperature.

Example 2: Preparation of multiple emulsion containing kojic acid in internal aqueous phase

Water-in-oil-in-water multiple emulsion was obtained through the same method of example 1 except that water-in-oil emulsion was prepared after 1wt% of kojic acid as a model of water-soluble active material had been introduced into internal aqueous phase. At this time, heat treatment has to be carefully monitored in order that the active material is not modified by heat.

Comparative example 1: Preparation of multiple emulsion with the conventional method

Conventional water-in-oil-in-water multiple emulsion was obtained through the same method of example 1 except that urea and hydroxypropyl-beta-cyclodextrine were not added. Multiple emulsions prepared by the conventional method and the present invention were used in the below experiments on stability thereof.

Experimental Example 1: Stability of multiple emulsion

The multiple emulsions obtained in Examples 1, 2 and Comparative Example 1 were used in the following experiments. In the meantime, all multiple emulsions used in this experiment were prepared after $MgSO_4$ was introduced into each internal aqueous phase of multiple emulsions. The obtained multiple emulsions were stored at 4°C, room temperature, 30°C, 37°C and 45°C, respectively. After a fixed amount of each multiple emulsion was taken once per day, conductivity was measured within unit of $1\,\mu S/cm$. Measured conductivity was compared with standard curves obtained by measuring conductivities depending on various concentrations of $MgSO_4$, and the stabilities of multiple emulsions were evaluated by the following formula.

$$\text{Stability of Mutiple Emulsion (\%)} = \left[ 1 - \frac{\text{Measured Conductivity (uS/cm)}}{\text{Calculated Conductivity (uS/cm)}} \right]$$

Stability of multiple emulsion obtained in Example 1 and Comparative Example 1 are evaluated relatively by measuring quantitatively the outflow of electrolytes in internal aqueous phase to external aqueous phase. As a result of relative evaluation on stability, it was found that about 15% of the electrolytes flowed out, immediately after preparing the multiple emulsion. It is thought that the initial outflow of electrolytes results from an unknown problem in the procedure re-emulsifying water-in-oil emulsion.

Stability of multiple emulsion obtained by HDS in Example 1 was maintained for several days at 70% or more, irrespective of storage conditions. However, the stability of multiple emulsion obtained by the conventional method decreased to less than 50%. This result proves that the procedure hydrophobizing internal aqueous phase hydrodynamically plays important roles in improving stability of multiple emulsion.

The multiple emulsion obtained in Example 2, which contains kojic acid in internal aqueous phase, was stored at 4°C, room temperature, 30°C, 37°C and 45°C, respectively. Then, changes of multiple emulsion in color, odor and titer were observed. Titer of multiple emulsion was determined through measuring concentration changes compared with initial concentration with HPLC. As a result, the original conditions of color and odor had been maintained for about two months. Also, result of titer test showed that 80% or more of initial titer had been maintained after one month.

Experimental Example 2: Analysis of multiple emulsion by using microscope

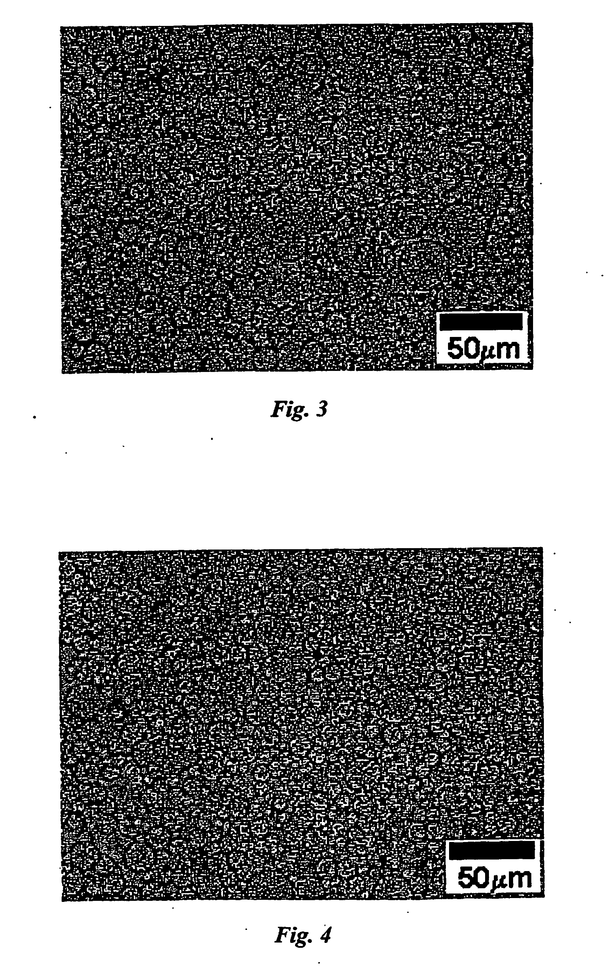

Droplet size and morphology of multiple emulsion obtained in Example 1 and Comparative Example 1 were measured using optical microscopic photographs, and the results are showed in Figures 2 and 3. As shown in optical microscopic photographs, both emulsions of Example 1 and Comparative Example 1 include two interfaces of water-in-oil-in-water. Also, when Figure 3 is compared with Figure 2, the multiple emulsion obtained by HDS has morphology of monolithic form (Figure 3), which is different from other conventional multiple emulsion. On the contrary, Figure 2 shows that using of general water forms a large internal aqueous phase. These results show that water in internal aqueous phase become dispersed to small size of droplets because interaction ability with oil phase is extremely improved through hydrophobizing the water in the internal aqueous phase. Also, the result measuring interfacial tension shows that in case of water introducing urea and hyroxypropyl-beta-cyclodextrine, interfacial tension was decreased sharply by 20 mN/m or more than pure water. This result indicates directly that reduction of polarity and aggregation of water may be achieved through introduction of urea and hydroxypropyl-beta-cyclodextrine.

Multiple emulsion stabilizing and containing 1wt% of kojic acid was observed with an optical microscope, and the result is shown in Figure 4. As shown in Figure 4, it is identified that morphology and droplet size were not modified, although large amount of water-soluble active material was added. Also, Figure 4 shows that water-soluble active material is positioned with extreme stability onto internal aqueous phase, and that reduction of emulsion stability during its preparation does not occur. In the meantime, droplet size of multiple emulsion is distributed evenly within the range of 2~20 μm. However, it is shown that the degree of dispersion was reduced a little when water in the internal aqueous phase was not treated with HDS. These results prove that the water in the internal aqueous phase passes through the oil phase, and intends to outflow to the external aqueous phase, thus the internal phase of multiple emulsion become instable.

As explained above, the present method for preparing multiple emulsion system through HDS is considerably different from other methods. The method according to the present invention may solve fundamental problems of unstable multiple emulsion systems of the conventional multiple emulsion system.

Therefore, the present invention may extend the conventional narrow use of multiple emulsion. In particular, when multiple emulsion prepared by the present HDS technology is applied to drug delivery systems, multiple emulsion according to the present invention may solve the problems, stability and activity of water-soluble active materials being sharply reduced in water. Also, multiple emulsion according to the present invention has excellent stability for storage and ability to block contact of internal aqueous phase and external aqueous phase. Therefore, the multiple emulsion system prepared by using HDS of the present invention may be effectively applied in various field including medical supplies and cosmetics.

Although preferred embodiments of the present invention have been described in detail hereinabove, it should be clearly understood that many variations and/or modifications of the basic inventive concepts herein taught which may appear to those skilled in the art will still fall within the spirit and scope of the present invention as defined in the appended claims.

**Claims**

1.    A water-in-oil-in-water multiple emulsion system stabilized by aggregating water molecules and diminishing polarity of the internal water phase; wherein the internal water phase comprises an agent for inhibiting hydrogen bonding which has mutual attraction in two or more directions to the hydrogen atoms or oxygen atom of a water molecule and an agent for aggregating water molecules which has an ability to conglomerate water molecules, wherein, said agent for inhibiting hydrogen bonding is selected from the group consisting of urea, thio-urea, alcohols with low molecular weight and amines with low molecular weight, and said agent for aggregating water molecules is selected from the group consisting of cyclodextrine and its derivatives.

2. A method for preparing the water-in-oil-in-water multiple emulsion system of claim 1, which comprises the following steps of:

(A) mixing water with an agent for inhibiting hydrogen bonding and an agent for aggregating water molecules;
(B) adding oil and a hydrophobic surfactant to the aqueous mixture prepared in step (A) and mixing homogeneously with mechanical shearing force to obtain stabilized water-in-oil emulsion;
(C) dropping the water-in-oil emulsion obtained in said step (B) into a water phase containing a hydrophilic surfactant, and re-emulsifying by stirring to obtain water-in-oil-in-water multiple emulsion; and,
(D) further stabilizing said multiple emulsion by adding a dispersion stabilizing agent to increase viscosity of external aqueous phase;

wherein said agent for inhibiting hydrogen bonding has mutual attraction in two or more directions to the hydrogen atoms or oxygen atom of a water molecule and is selected from the group consisting or urea, thio-urea, alcohols with low molecular weight and amines with low molecular weight; and said agent for aggregating water molecules has an ability to conglomerate water molecules and is selected from the group consisting of cyclodextrine and its derivatives.

3. The method according to claim 2, wherein HLB value of said hydrophobic surfactant is within the range of 1-7, and said hydrophilic surfactant is at least one selected from non-ionic surfactant within the HLB range of 8~20 and ionic surfactant within the HLB range of 20 or more.

4. The method according to claim 2, wherein said dispersion stabilizing agent is a macromolecule dissolved in the water phase in part or completely, is selected from the group consisting of gelatin, starch, xanthan gum, sodium polyacrylate, hydroxyethyl cellulose, carboxymethyl cellulose, polyvinylpyrrolidone, polyvinyl alkyl ethers, polyvinyl alcohols, random copolymers and block copolymers conjugated hydrophilic group to main chain.

5. The method according to claim 2, wherein said hydrogen bonding inhibitor is 0.01~5% by weight based on total weight of the water-in-oil-in-water multiple emulsion system, water molecule aggregating agent is 0.01~5% by weight based on the total weight of the water-in-oil-in-water multiple emulsion system; hydrophobic surfactant is 0.01~10% by weight on total weight, hydrophilic surfactant is 0.01~10% by weight based on the total weight of the water-in-oil-in-water multiple emulsion system; and, dispersion stabilizing agent is 0.01~10% by weight based on the total weight of the water-in-oil-in-water multiple emulsion system.

6. A cosmetic composition comprising at least one water-soluble active component selected from the group consisting of vitamin B and its derivatives, vitamin C and its derivatives, kojic acid and its derivatives, peptide compounds, proteins, enzymes, water-soluble plant extracts and water-soluble functional materials, wherein said water-soluble active component is contained in the internal water phase of the water-in-oil-in-water multiple emulsion system of claim 1.

7. A medical composition comprising at least one water-soluble active component selected from the group consisting of amino acids and water-soluble antibiotics, wherein said water-soluble active component is contained in the internal water phase of the water-in-oil-in-water multiple emulsion system of claim 1.

**Patentansprüche**

1. Multiples Wasser-in-Öl-in-Wasser-Emulsionssystem, das durch ein Aggregieren von Wassermolekülen und ein Vermindern der Polarität der inneren Wasserphase stabilisiert ist, wobei die innere Wasserphase ein Mittel zum Hemmen der Wasserstoffbindung, das ein gegenseitiges Anziehungsvermögen in zwei oder mehreren Richtungen bezüglich der Wasserstoffatome oder des Sauerstoffatoms eines Wassermoleküls aufweist, und ein Mittel zum Aggregieren von Wassermolekülen aufweist, das eine Fähigkeit zur Bildung eines Konglomerats der Wassermoleküle aufweist, und wobei das Mittel zum Hemmen der Wasserstoffbindung aus der Gruppe ausgewählt ist, die aus Harnstoff, Thioharnstoff, Alkoholen mit niedrigem Molekulargewicht und Aminen mit niedrigem Molekulargewicht besteht, und das Mittel zum Aggregieren der Wassermoleküle aus der Gruppe ausgewählt ist, die aus Cyclodextrin und seinen Derivaten besteht.

**2.** Verfahren zur Herstellung des multiplen Wasser-in-Öl-in-Wasser-Emulsionssystems nach Anspruch 1, das die folgenden Schritte umfasst:

(A) Mischen von Wasser mit einem Mittel zum Hemmen der Wasserstoffbindung und einem Mittel zum Aggregieren von Wassermolekülen,
(B) Hinzufügen von Öl und von einem hydrophoben grenzflächenaktiven Stoff zu dem in dem Schritt (A) präparierten wässerigen Gemisch und homogenes Mischen mit einer mechanischen Scherkraft, um eine stabilisierte Wasser-in-Öl-Emulsion zu erhalten,
(C) Tropfen der im Schritt (B) erhaltenen Wasser-in-Öl-Emulsion in eine Wasserphase, die einen hydrophilen grenzflächenaktiven Stoff enthält, und Rück-Emulgieren durch Rühren, um eine multiple Wasser-in-Öl-in-Wasser-Emulsion zu erhalten, und
(D) weiteres Stabilisieren der multiplen Emulsion durch Hinzufügen eines Dispersionsstabilisierungsmittels, um die Viskosität der externen wässerigen Phase zu erhöhen,

wobei das Mittel zum Hemmen der Wasserstoffbindung ein gegenseitiges Anziehungsvermögen in zwei oder mehreren Richtungen bezüglich der Wasserstoffatome oder des Sauerstoffatoms eines Wassermoleküls aufweist und aus der Gruppe ausgewählt ist, die aus Harnstoff, Thioharnstoff, Alkoholen mit niedrigem Molekulargewicht und Aminen mit niedrigem Molekulargewicht besteht, und das Mittel zum Aggregieren der Wassermoleküle eine Fähigkeit zur Bildung eines Konglomerats der Wassermoleküle aufweist und aus der Gruppe ausgewählt ist, die aus Cyclodextrin und seinen Derivaten besteht.

**3.** Verfahren nach Anspruch 2, worin der HLB-Wert des hydrophoben grenzflächenaktiven Stoffs im Bereich von 1~7 liegt und der hydrophile grenzflächenaktive Stoff zumindest einer ist, der aus einem nichtionischen grenzflächenaktiven Stoff innerhalb des HLB-Bereichs von 8~20 und einem ionischen grenzflächenaktiven Stoff innerhalb des HLB-Bereichs von 20 oder mehr ausgewählt ist.

**4.** Verfahren nach Anspruch 2, worin das Dispersionsstabilisierungsmittel ein Makromolekül ist, das in der Wasserphase teilweise oder vollständig gelöst ist, und aus der Gruppe ausgewählt ist, die aus Gelatine, Stärke, Xanthan Gum, Natriumpolyacrylat, Hydroxyethylzellulose, Karboxymethylzellulose, Polyvinylpyrrolidon, Polyvinylalkylether, Polyvinylalkoholen, statistischen Kopolymeren und Blockkopolymeren, die eine an der Hauptkette konjugierte hydrophile Gruppe aufweisen, besteht.

**5.** Verfahren nach Anspruch 2, worin der Wasserstoffbindungshemmer auf der Basis des Gesamtgewichts des multiplen Wasser-in-Öl-in-Wasser-Emulsionssystems 0,01~5 Gewichtsprozent ausmacht, das Wassermolekülaggregationsmittel auf der Basis des Gesamtgewichts des multiplen Wasser-in-Öl-in-Waser-Emulsionssystems 0,01~5 Gewichtsprozent ausmacht, der hydrophobe grenzflächenaktive Stoff 0,01~10 Gewichtsprozent des Gesamtgewichts ausmacht, der hydrophile grenzflächenaktive Stoff 0,01~10 Gewichtsprozent auf der Basis des Gesamtgewichts des multiplen Wasser-in-Öl-in-Wasser-Emulsionssystems ausmacht und das Dispersionsstabilisierungsmittel 0,01~10 Gewichtsprozent auf der Basis des Gesamtgewichts des multiplen Wasser-in-Öl-in-Wasser-Emulsionssystems ausmacht.

**6.** Kosmetische Zusammensetzung mit zumindest einer wasserlöslichen aktiven Komponente, die aus der Gruppe ausgewählt ist, die aus Vitamin B und seinen Derivaten, Vitamin C und seinen Derivaten, Kojisäure und ihren Derivaten, Peptidverbindungen, Proteinen, Enzymen, wasserlöslichen Pflanzenextrakten und wasserlöslichen funktionellen Materialien besteht, worin die wasserlösliche aktive Komponente in der inneren Wasserphase des multiplen Wasser-in-Öl-in-Wasser-Emulsionssystems nach Anspruch 1 enthalten ist.

**7.** Medizinische Zusammensetzung mit zumindest einer wasserlöslichen aktiven Komponente, die aus der Gruppe ausgewählt ist, die aus Aminosäuren und wasserlöslichen Antibiotika besteht, worin die wasserlösliche aktive Komponente in der inneren Wasserphase des multiplen Wasser-in-Öl-in-Wasser-Emulsionssystems nach Anspruch 1 enthalten ist.

**Revendications**

**1.** Un système d'émulsion multiple eau/huile/eau stabilisée en agrégeant des molécules d'eau et en diminuant la polarité de la phase eau interne ; dans lequel la phase eau interne comprend un agent d'inhibition de liaison hydrogène qui a une attraction mutuelle dans deux directions ou plus vers les atomes d'hydrogène ou l'atome d'oxygène d'une molécule d'eau et un agent d'agrégation de molécules d'eau qui a une capacité d'agglomérer des molécules d'eau, dans lequel ledit agent d'inhibition de liaison hydrogène est choisi dans le groupe constitué par l'urée, la thio-urée, des alcools à faible poids moléculaire et des amines à faible poids moléculaire et ledit agent d'agrégation de molécules d'eau est choisi dans le groupe constitué par la cyclodextrine et ses dérivés.

2. Un procédé pour préparer le système d'émulsion multiple eau/huile/eau de la revendication 1 qui comprend les étapes suivantes de :

(A) mélanger de l'eau avec un agent d'inhibition de liaison hydrogène et un agent d'agrégation de molécules d'eau ;
(B) ajouter de l'eau et un agent de surface hydrophobe au mélange aqueux préparé à l'étape (A) et mélanger de manière homogène avec une force de cisaillement mécanique à l'émulsion eau/huile stabilisée obtenue ;
(C) faire tomber goutte à goutte l'émulsion eau/huile obtenue dans ladite étape (B) dans une phase eau contenant un agent de surface hydrophile et ré-émulsfier en agitant pour obtenir une émulsion multiple eau/huile/eau ; et
(D) stabiliser à nouveau ladite émulsion multiple en ajoutant un agent de stabilisation de dispersion pour augmenter la viscosité de la phase aqueuse externe ;

dans lequel ledit agent d'inhibition de liaison hydrogène a une attraction mutuelle dans deux directions ou plus vers les atomes d'hydrogène ou l'atome d'oxygène d'une molécule d'eau et est choisi dans le groupe constitué par l'urée, la thio-urée, des alcools à faible poids moléculaire et des amines à faible poids moléculaire ; et ledit agent d'agrégation de molécules d'eau a une capacité d'agglomérer des molécules d'eau et est choisi dans le groupe constitué par la cyclodextrine et ses dérivés.

3. Le procédé selon la revendication 2,
dans lequel la valeur HLB dudit agent de surface hydrophobe est comprise dans la plage de 1-7 et ledit agent de surface hydrophile est au moins un agent choisi parmi un agent de surface non-ionique à l'intérieur de la plage HLB de 8-20 et un agent de surface ionique à l'intérieur de la plage HLB de 20 ou plus.

4. Le procédé selon la revendication 2,
dans lequel ledit agent de stabilisation de dispersion est une macromolécule dissoute dans la phase eau partiellement ou complètement, est choisi dans le groupe formé par la gélatine, l'amidon, la gomme de xanthane, le polyacrylate de sodium, l'hydroxyéthylcellulose, la carboxyméthylcellulose, la polyvinylpyrrolidone, les polyvinylalkyléthers, les polyvinylalcools, un groupe hydrophile conjugué à des copolymères aléatoires et des copolymères en masse alcaline à la chaîne principale.

5. Le procédé selon la revendication 2,
dans lequel ledit inhibiteur de liaison hydrogène est de 0,01-5 % en poids sur la base du poids total du système d'émulsion multiple eau/huile/eau, l'agent d'agrégation de molécules d'eau est de 0,01-5 % en poids sur la base du poids total du système d'émulsion multiple eau/huite/eau, l'agent de surface hydrophobe est de 0,01-10 % en poids du poids total, l'agent de surface hydrophile est de 0,01-10 % en poids sur la base du poids total du système d'émulsion multiple eau/huile/eau et l'agent de stabilisation de dispersion est de 0,01-10 % en poids sur la base du poids total du système d'émulsion multiple eau/huile/eau.

6. Une composition cosmétique comprenant au moins un composant actif soluble dans l'eau choisi dans le groupe constitué par la vitamine B et ses dérivés, la vitamine C et ses dérivés, l'acide cogique et ses dérivés, des composés de peptides, des protéines, des enzymes, des extraits de plantes solubles dans l'eau et des matériaux fonctionnels solubles dans l'eau, dans laquelle ledit composant actif soluble dans l'eau est contenu dans la phase eau interne du système d'émulsion multiple eau/huile/eau de la revendication 1.

7. Une composition médicale comprenant au moins un composant actif soluble dans l'eau choisi dans le groupe constitué par des acides aminés et des antibiotiques solubles dans l'eau, dans laquelle ledit composant actif soluble dans l'eau est contenu dans la phase eau interne du système d'émulsion multiple eau/huile/eau de la revendication 1.

# *Figure*

Low shear homogenization

Water phase | Oil phase

HP–β–CD
Arlacel P135
Water molecule

*Fig. 1*

50μm

*Fig. 2*

*Fig. 3*

*Fig. 4*